Europäisches Patentamt

European Patent Office

Office européen des brevets

(19)

(11) Veröffentlichungsnummer: **0 131 541**
**B1**

(12) **EUROPÄISCHE PATENTSCHRIFT**

(45) Veröffentlichungstag der Patentschrift: **27.12.90**

(51) Int. Cl.⁵: **C 12 M 1/00,** B 01 L 1/02

(21) Anmeldenummer: **84810252.1**

(22) Anmeldetag: **24.05.84**

(54) **Begasungs-Brutschrank für biologische Anwendung.**

(30) Priorität: **13.06.83 CH 3238/83**
**21.03.84 CH 1425/84**

(43) Veröffentlichungstag der Anmeldung:
**16.01.85 Patentblatt 85/03**

(45) Bekanntmachung des Hinweises auf die
Patenterteilung:
**27.12.90 Patentblatt 90/52**

(84) Benannte Vertragsstaaten:
**DE FR GB IT NL SE**

(56) Entgegenhaltungen:
**DE-A-2 721 862**
**FR-A-2 179 506**
**GB-A- 333 434**
**GB-A- 926 541**

(73) Patentinhaber: **SALVIS AG**
**Hauptstrasse 49**
**CH-6015 Reussbühl (CH)**

(72) Erfinder: **Balmer, Willi**
**Sagenstrasse 4c**
**CH-6030 Ebikon (CH)**
Erfinder: **Süess, Josef**
**Spitzberglistrasse 4**
**CH-6048 Horw (CH)**
Erfinder: **Kull, Fritz**
**Kilchliried 1**
**CH-6362 Stansstad (CH)**

(74) Vertreter: **Blum, Rudolf Emil Ernst et al**
**c/o E. Blum & Co Patentanwälte Vorderberg 11**
**CH-8044 Zürich (CH)**

Courier Press, Leamington Spa, England.

**Beschreibung**

In der Biotechnologie arbeitet man mit Mikroorganismen und Zellkulturen, welche im Labormassstab in begasten Brutschränken gezüchtet werden. Im Nutzraum solcher Brutschränke für biologische Anwendung werden nebst einer bestimmten, möglichst konstanten Temperatur und Feuchtigkeit Gase über mindestens eine Leitung zugeführt. Bei Schränken älterer Bauart wird das zuführende Gas - meistens Kohlendioxid - ausserhalb des Nutzraumes einem konstanten Luftstrom über Regulierventile und Durchflussmesser beigemischt und in den Nutzraum eingeblasen. Diese Art von Gaszufuhr ergibt zwar eine gute Durchmischung des zugeführten Gas-Luftgemisches, jedoch muss dauernd eine überschüssige Gasmenge zugeführt werden, um Störgrössen im Nutzraum, beispielsweise durch Oeffnen der Tür, auszugleichen.

Um derartige kostspielige Gasverluste zu vermeiden, wird bei Schränken neuerer Bauart ein Regelsystem eingesetzt, das den Gasgehalt im Nutzraum misst und über Stellorgane fehlende Gasanteile zuführt. Da die zugeführten, reinen Gase eine andere Dichte haben, als das sich bereits im Nutzraum befindliche gasförmige Medium, kann dies zu Schichtbildungen der Gasphasen und damit zu Fehlmessungen im Regelsystem führen. Praktische Laborerfahrungen bestätigen dies.

Es ist bereits vorgeschlagen worden, diese Nachteile dadurch zu beheben, dass seitlich an der Wand oder an der Schrankdecke Ventilatoren angebracht wurden, welche das Gasgemisch im Nutzraum umwälzen und durchmischen sollen. Es kann hierzu auf die Publikation GB-A 926 541 verwiesen werden.

Im Nutzraum solcher Brutschränke wachsen erfahrungsgemäss nach einigen Tagen auch ausserhalb der eingesetzten Brutgefässe Mikroorganismen in Form von Pilzen und Bakterien, welche u.U. die Resultate der Brut verfälschen können. Ein wichtiger Vorgang ist daher die periodische Reinigung des Nutzraumes und dessen Desinfektion. Dieser Reinigungsvorgang ist umso wirksamer, je weniger schwerzugängliche Ecken, Ritzen, Kabel, Messelemente, Dichtungen oder dgl. im Nutzraum vorhanden sind. Der reinigungstechnische Idealfall wäre ein Nutzraum ohne Durchbrüche und glatten Innenwandflächen mit gerundeten Ecken.

Diesem Idealfall steht die Forderung entgegen, dass im Innern des Brutschrankes Elektro- und Gasleitungen, Ventilatoren, Heizelemente und andere Funktionselemente anzuordnen sind, um eine gewünschte gleichmässige Begasung und allenfalls Heizung und Beeinflussung der Atmosphäre im Innenraum des Brutschrankes vornehmen zu können. Diese Durchbrüche und Einbauten erschweren indessen eine einfache Reinigung, zumal die Zuleitungen, Halterungen, Fühler und dgl. üblicherweise noch mit Schutzblechen und Blenden verdeckt sind, welche den Zugang erschweren.

Es stellt sich deshalb die Aufgabe, einen Begasungs-Brutschrank der eingangs genannten Art so zu verbessern, dass er diese Nachteile nicht aufweist.

Diese Aufgabe wird durch die im Kennzeichen von Anspruch 1 genannten Merkmale gelöst.

Dabei ist es vorteilhaft, wenn das Standrohr mit dem Lüfterrad für die Umwälzung und weiteren Funktionsorganen, wie Mittel zur Befeuchtung, Heizung, Messung oder Regulierung in einem vom Nutzraum trennbaren Gehäuse untergebracht sind, wodurch das Innere von Einbauten und Durchbrüchen frei gehalten werden kann.

In der Zeichnung sind Ausführungsbeispiele des Erfindungsgegenstandes dargestellt. Es zeigen:

Fig. 1 eine perspektivische Ansicht einer Ausführungsvariante eines Begasungs-Brutschrankes von vorne, bei entfernter Fronttür;

Fig. 2 einen vertikalen Querschnitt durch den Schrank;

Fig. 3 einen Schnitt durch den Brutschrank mit eingebautem Gehäuse, nach der Linie VI-VI in Fig. 4;

Fig. 4 eine Vorderansicht des Gehäuses gemäss Fig. 3.

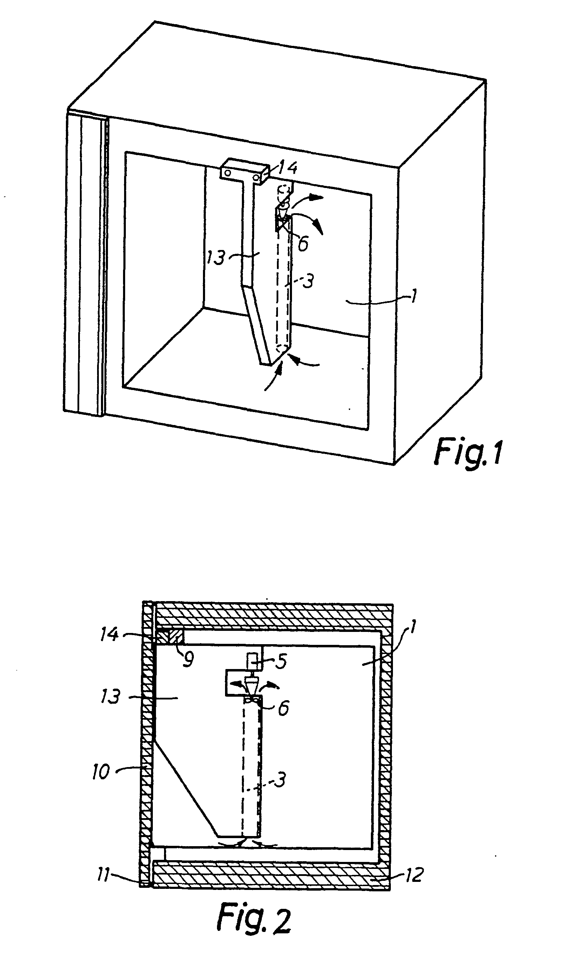

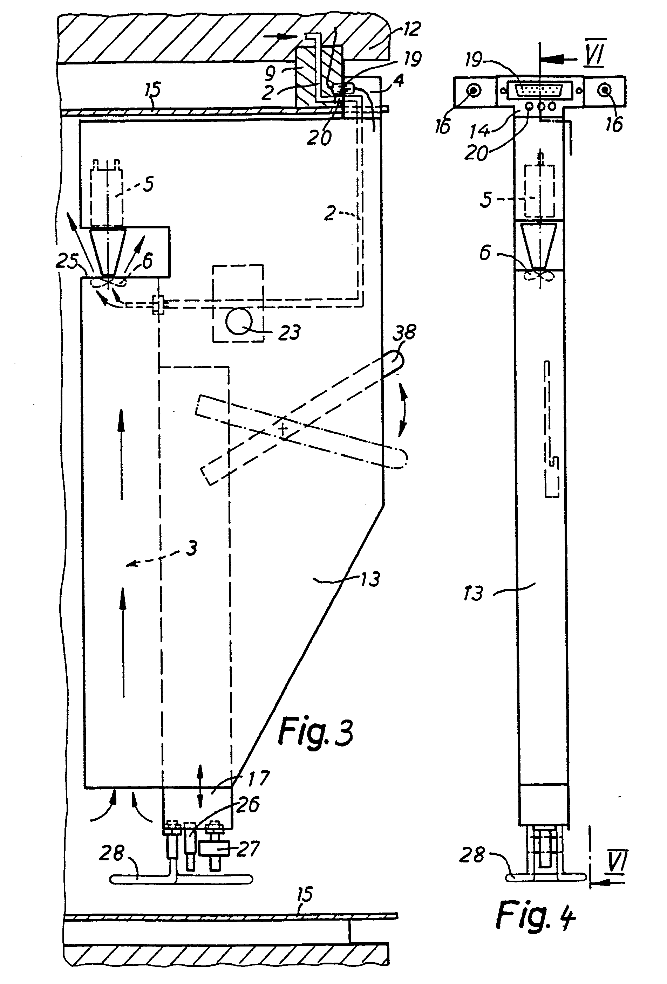

Der Begasungs-Brutschrank gemäss den Fig. 1 bis 4 ist von einem wärmeisolierenden Material 12 umgeben, welches einen etwa quaderförmigen Nutzraum 1 umschliesst, der durch eine metallische Schale 15 begrenzt ist. Die Vorderseite ist durch eine schwenkbare Tür 10 abschliessbar, wobei ein luftdichter Abschluss mittels einer ringsherumlaufenden Dichtung 11 erreicht wird. Ein Gehäuse 13 ragt in den Mittelbereich dieses Nutzraumes 1 hinein und ist oben an einem schienenartigen Tragblock 9 aufgehängt. Dieser Tragblock 9 ist oben mit dem Schrank 1 starr verbunden und enthält elektrische und mechanische Kupplungsstücke 19, 20, um sowohl gasförmige Medien als auch elektrische Leiter dem Gehäuse 13 zuführen zu können.

Dieser Tragblock 9 ist an der Oberseite des Innenraumes 2 nahe bei seiner frontseitigen Oeffnung mit dem Brutschrank fest angeordnet. Am Gehäuse 13 befindet sich eine mit dem Tragblock 9 zusammenwirkende Kupplungsleiste 14, so dass das Gehäuse 13 von Hand leicht in Horizontalrichtung aus dem Innenraum 2 herausgezogen bzw. in diesen eingesetzt werden kann.

Zwischen dem Tragblock 9 und der Kupplungsleiste 14 ist ein trennbarer elektrischer Stecker 19 und eine trennbare Rohrkupplung 20 vorhanden. Zur Lagesicherung können entweder Zentrierstifte 16 oder Gewindebolzen vorhanden sein.

Im Innern des Gehäuses 13 befindet sich als Teil desselben - ein vertikales Standrohr 3, das unten einen Abstand vom Boden des Innenraumes hat. Im oberen Bereich des vom Standrohr 3 umgebenen Kanales ist ein Kleinmotor 5 angeordnet, der ein Axiallüfterrad 6 antreibt. Das durch dieses Lüfterrad 6 von unten nach oben geförderte gasförmige Medium verlässt den Kanal bei einer Oeffnung 25 im Bereich des oberen Innenraumendes. Dadurch entsteht im Innern des Nutzraumes eine schwache Gasbewegung und erzeugt eine Durchmischung des gasförmigen Mediums. Auf diese Weise wird zudem eine gleichmässige Temperaturverteilung erreicht und eine Gasschichtbildung vermieden. Sämtliche für die Regulierung und Sicherheit benötigten mechanischen und elektrischen Mess- und Regulierorgane sind in diesem Gehäuse 13 untergebracht. Ausserdem ist unten an einem vertikal beweglichen Hubwerk 17 ein Feuchteerzeuger mit einer elektrischen Wasserheizung 28, einem Wasser-Niveaufühler 27 und einem Temperaturfühler 26 befestigt. Zum Heben und Absenken des Hubwerkes 17 ist eine Hebelvorrichtung 38 vorhanden, um den Feuchteerzeuger in ein nicht eingezeichnetes schalenförmiges Wasserreservoir eintauchen oder aus diesem herausheben zu können. Ausserdem können im Gehäuse in der Zeichnung nicht eingezeichnete Messleitungen für externe Probenentnahmen vorhanden sein. In der Nutzraummitte befindet sich etwa auf halber Höhe im Gehäuse 13 ein Gasmessfühler 23 zur schnellen und repräsentativen Feststellung von Messwerten.

Eine von aussen speisbare Gaszufuhrleitung 2 mündet über das Kupplungsstück 19 und ein sich im Innern des Gehäuses 13 befindliches Rohr knapp unterhalb des Axiallüfters 6 in das Standrohr 3 ein.

Das Gehäuse 13 samt allen Einbauten wird vorzugsweise aus Materialien hergestellt, die Sterilisations-Temperaturen aushalten. Dadurch ist es möglich, dieses Gehäuse 13 aus dem Innenraum herauszuziehen und samt allen Einbauten in üblicher Weise in einem Autoklav oder dgl. zu sterilisieren.

Die elektrische Heizung kann allenfalls aussen um die Wandung des Nutzraumes herum angeordnet werden. Eine Zusatzheizung kann allenfalls trotzdem im Gehäuse 13 vorgesehen werden.

Von den oben ausgeführten, der Bewegung des gasförmigen Mediums, Befeuchtung, Messung, Regulierung und Heizung dienenden Organen könnten allenfalls nicht alle sondern nur ein Teil derselben vorhanden und im Gehäuse 13 eingebaut sein.

**Patentansprüche**

1. Begasungs-Brutschrank mit einem Nutzraum, der vorderseitig durch eine Tür dicht abschliessbar ist, wobei Umwälzmittel für die Bewegung eines gasförmigen Mediums im Nutzraum vorgesehen sind, welche einen stehend angeordneten Kanal (3) mit unteren und oberen Oeffnungen sowie ein darin angeordnetes, antreibbares Lüfterrad aufweisen, dadurch gekennzeichnet, dass die Umwälzmittel mit ihren Zuleitungen in einem von der Schrankvorderseite her einsetzbaren bzw. herausnehmbaren Traggehäuse (13) untergebracht sind, das durch Kupplungsorgane (19, 20) im Türrahmenbereich ausserhalb des Nutzraums mit dem Schrank trennbar verbunden ist und von der Vorderseite her in den Nutzraum ragt.

2. Begasungs-Brutschrank nach Anspruch 1, dadurch gekennzeichnet, dass am Traggehäuse (13) ausser den Umwälzmitteln weitere Funktionsorgane, wie Mittel zur Befeuchtung, Heizung, Messung und/oder Regulierung sowie deren Zuleitungen angeordnet sind, wobei die Kupplungsorgane Anschlüsse für die Zuleitungen aller Funktionsorgane aufweisen.

3. Begasungs-Brutschrank nach einem der Ansprüche 1 bis 7, dadurch gekennzeichnet, dass in den Kanal (3) ein Gaseinlassrohr (2) einmündet.

4. Begasungs-Brutschrank nach einem der Ansprüche 1 bis 3, dadurch gekennzeichnet, dass des Traggehäuse (13) ausserhalb der Innenraum-Decke hängend ausgebildet ist.

5. Begasungs-Brutschrank nach einem der Ansprüche 1 bis 4, dadurch gekennzeichnet, dass der stehend angeordnete Kanal (3) mindestens unten stirnseitig offen und vom Nutzraumboden (10) distanziert angeordnet ist.

6. Begasungs-Brutschrank nach einem der Ansprüche 1 bis 5, dadurch gekennzeichnet, dass sich der stehend angeordnete Kanal (3) im mittleren Nutzraumbereich befindet.

7. Begasungs-Brutschrank nach einem der Ansprüche 1 bis 6, dadurch gekennzeichnet, dass das Volumen des Kanals (3) zum Volumen des gesamten Nutzraumes klein ist, z.B. in der Grössenordnung von 1: 200 bis 1: 600, vorzugsweise etwa 1: 400.

8. Begasungs-Brutschrank nach einem der Ansprüche 1 bis 7, dadurch gekennzeichnet, dass die Kupplungsorgane mindestens eine trennbare Rohrkupplung (20) und mindestens eine lösbare elektrische Steckerverbindung (19) umfassen.

9. Begasungs-Brutschrank nach einem der Ansprüche 1 bis 8, dadurch gekennzeichnet, dass das Traggehäuse (13) samt Einbauten aus Materialien bestehen, welche Sterilisations-Temperaturen standhalten.

10. Begasungs-Brutschrank nach einem der Ansprüche 1 bis 9, dadurch gekennzeichnet, dass das Traggehäuse (13) oben an seiner vorderen Seite eine Kupplungsleiste (14) besitzt und im Türrahmenbereich aussen an der Deckwand des Nutzraums ein entsprechender Tragblock (9) angeordnet ist, welche die Kupplungsorgane sowie Mittel (16) zur Lagesicherung des Traggehäuses enthalten.

11. Begasungs-Brutschrank nach einem der Ansprüche 1 bis 9 dadurch gekennzeichnet, dass das Innere des Nutz-raums frei von Durchbrüchen gehalten ist.

**Revendications**

1. Etuve bactériologique à circulation de gaz comprenant un espace utile, qui peut être hermétiquement fermé sur le devant au moyen d'une porte, des moyens de circulation d'un milieu gazeux étant prévus dans l'espace utile, lesquels présentent un canal (3) vertical, pourvu d'ouvertures inférieures et supérieures ainsi qu'à l'intérieur de celui-ci, une roue de ventilateur pouvant être entraînée, caractérisée en ce que les moyens de circulation et leurs conduites d'alimentation sont logés dans un boîtier porteur (13) pouvant être introduit ou sorti par l'avant de l'étuve et étant relié de façon amovible à l'étuve, au niveau de l'encadrement de la porte, à l'extérieur de l'espace utile, au moyen d'organes de couplage (19, 20), et qui est en saillie dans l'espace utile à partir de l'avant.

2. Etuve bactériologique à circulation de gaz suivant la revendication 1, caractérisée en ce qu'en plus des moyens de circulation, d'autres organes fonctionnels, tels que des moyens servant à l'humidification, au chauffage, à la mesure et/ou au réglage ainsi que les conduites d'alimentation de ceux-ci sont montés sur le boîtier porteur (13), les organes de couplage présentent des raccordements pour les conduites d'alimentation de tous les organes fonctionnels.

3. Etuve bactériologique à circulation de gaz suivant une des revendications 1 à 2, caractérisée en ce qu'un tuyau d'alimentation de gaz (2) débouche dans le canal (3).

4. Etuve bactériologique à circulation de gaz suivant une des revendications 1 à 3, caractérisée en ce que le boîtier porteur (13) est réalisé de manière suspendue sous le plafond de la chambre interne.

5. Etuve bactériologique à circulation de gaz suivant une des revendications 1 à 4, caractérisée en ce que le canal vertical (3) est ouvert au moins en bas sur sa face frontale à une certaine distance du fond de l'espace utile (10).

6. Etuve bactériologique à circulation de gaz suivant une des revendications 1 à 5, caractérisée en ce que le canal vertical (3) se trouve dans la zone centrale de l'espace utile.

7. Etuve bactériologique à circulation de gaz suivant une des revendications 1 à 6, caractérisée en ce que le volume du canal (3) est faible par rapport au volume de l'ensemble de l'espace utile, par exemple de l'ordre de grandeur de 1: 200 à 1: 600 et de préférence de 1: 400.

8. Etuve bactériologique à circulation de gaz suivant une des revendications 1 à 7, caractérisée en ce que les organes de couplage comprennent au moins un raccord de tuyaux séparable (20) et au moins une connexion électrique par fiche 19 débranchables.

9. Etuve bactériologique à circulation de gaz suivant une des revendications 1 à 8, caractérisée en ce que le boîtier porteur (13), ainsi que l'ensemble des éléments incorporés, sont réalisés en une matière résistant aux températures de stérilisation.

10. Etuve bactériologique à circulation de gaz suivant une des revendications 1 à 9, caractérisée en ce que le boîtier porteur (13) possède, en haut et devant une barrette de couplage (14) et qu'à l'extérieur, au niveau de l'encadrement de la porte, un bloc porteur (9) est monté sur la face supérieure de l'espace utile, qui contient les organes de couplage ainsi que des moyens (16) permettant de consolider la position du boîtier porteur.

11. Etuve bactériologique à circulation de gaz suivant une des revendications 1 à 9, caractérisée en ce que l'intérieur de l'espace utile reste libre de toute interruption.

## Claims

1. Biological incubator with gas circulation, having a useful space hermetically closable at its front side by means of a door, whereas circulation means are provided for circulating a gas medium within said useful space, comprising an upright channel (3) having a lower and an upper opening and a drivable fan wheel arranged therein, characterized in that the circulation means together with their supply lines are placed in a support housing (13) insertable and removable from the front side of the incubator, which is removably connected to the incubator by means of coupling members (19, 20) arranged outside of the useful space at the door frame zone and which projects from the front side into the useful space.

2. Biological incubator of claim 1, characterized in that in addition to the circulation means further functional members, as moistening means, heating means, measuring means and/or control means and their supply lines are arranged at the support housing, whereas the coupling members comprise connections for the supply lines of all functional members.

3. Biological incubator of one of the claims 1 to 2, characterized in that a gas inlet tube (2) opens into the channel (3).

4. Biological incubator of one of the claims 1 to 3, characterized in that the support housing (13) is suspendedly arranged outside of the cover wall of the inside space.

5. Biological incubator of one of the claims 1 to 4, characterized in that the upright channel (3) is open at least at its lower front end and arranged in a distance from the bottom wall (10) of the useful space.

6. Biological incubator of one of the claims 1 to 5 characterized in that the upright channel (3) is located in the center zone of the useful space.

7. Biological incubator of one of the claims 1 to 6 characterized in that the volume of the channel (3) is small compared with the volume of the whole useful space, e.g. in a range of 1:200 to 1:600, preferably about 1:400.

8. Biological incubator of one of the claims 1 to 7, characterized in that the coupling members comprise at least a separable tube coupling (20) and at least a releasable electric plug connection (19).

9. Biological incubator of one of the claims 1 to 8 characterized in that the support housing (13) and its components consist of materials which withstand sterilization temperatures.

10. Biological incubator of one of the claims 1 to 9, characterized in that the support housing (13) at its upper front side comprises a coupling ledge (14) and that in a corresponding supporting block (9) is arranged in the door frame zone at the outside of the cover wall of the useful space, comprising the coupling members as well as

EP 0 131 541 B1

members (16) for securing the position of the support housing.

11. Biological incubator of one of the claim 1 to 9, characterized in that the inside of the useful space is free of punctures.

Fig.1

Fig. 2

Fig. 3

Fig. 4